# EUROPEAN PATENT SPECIFICATION

(11) **EP 0 498 801 B1**
(45) Date of publication and mention of the grant of the patent: **04.11.1998**
(21) Application number: 90913430.6
(22) Date of filing: 13.09.1990
(51) Int. Cl.: C07K 1/04

(54) **SOLID SURFACE FOR PEPTIDE SYNTHESIS**
FESTES SUBSTRAT FÜR PEPTIDSYNTHESE
SURFACE SOLIDE POUR LA SYNTHESE DE PEPTIDES

(30) Priority: 15.09.1989 AU 6383/89
(43) Date of publication of application: 19.08.1992
(73) Proprietor: CHIRON MIMOTOPES PTY. LTD., Clayton, VIC 3168 (AU)
(72) Inventor: GEYSEN, Hendrik, Mario, Menzies Creek, VIC 3159 (AU)
(74) Representative: Goldin, Douglas Michael
(86) International application number: AU9000416
(87) International publication number: WO9104266

(56) References cited:
- WO-A-84/02526
- AU-A- 2 542 984
- AU-A- 3 869 289
- AU-A- 4 215 389
- AU-A- 7 645 687
- AU-B- 1 226 088
- DE-A- 3 723 004
- US-A- 3 557 077
- US-A- 4 515 920

## Description

This invention relates to a solid surface for the synthesis of peptides thereon, and in particular relates to a rod or pin having a surface on which a peptide may be synthesized efficiently and economically.

In prior International Patent Specification No. PCT/AU84/00039, there is disclosed a method for the simultaneous synthesis of a large number of different peptides. Basically, this method involves the synthesis of peptides onto a solid polymeric surface, such as polyethylene, which is molded into the shape of a rod or pin. In a preferred embodiment of the method (but not limited to it), these rods or pins are positioned in a holder so that they form a 12 by 8 matrix, with the rods or pins being positioned so that the spacing corresponds to that of the wells of microtitre plates which are widely used for ELISA (enzyme-linked immunosorbent assay) tests.

The method disclosed in this prior specification was based on the realization that for the solid-phase synthesis of any peptide, almost all of the steps of the synthesis are exactly the same for any peptide and are independent of the sequence of the peptide being synthesized. Thus, with the rods or pins arranged in the preferred format so that 96 are in a holder, all steps in the synthesis such as washing steps, neutralization steps and deprotection steps can be carried out simultaneously in the synthesis of 96 different peptides. The only steps which must be carried out separately for each different peptide are the coupling of the appropriate amino acid residues. Each of these steps is conveniently carried out by dispensing appropriate activated amino acid solution into the corresponding wells of a microtitre plate or the like. Thus, the appropriate amino acid is coupled to the peptide for each of the rods.

The quantity of peptide covalently bonded to the solid polymer surface by this method is sufficient to allow reaction of the peptide with specific binding entities such as antibodies to be readily detected. Although the quantity of peptide synthesized on each rod is relatively small (being of the order of tens of nanomoles), the ability to reuse the synthesized peptide after a test compensates for the small quantity of peptide on the rod. However, in some applications the quantity of peptide needs to be greater. Examples of such applications include the removal of the peptide from the rod and recovery of specific binding entities for further testing. Accordingly, modifications to the process of synthesis and testing of the peptides have been disclosed in Australian Patent Specification No. PJ2788/89.

It is an object of the present invention to provide means by which the amount of peptide, or for that matter any other polymeric compound such as nucleic acid which can be synthesized on a solid support, can be increased while retaining the advantage of being able to synthesize many peptides with different sequences simultaneously.

In the earlier method disclosed in International Patent Specification No. PCT/AU84/00039 polyacrylic acid was grafted to the surface of a solid polyethylene support using gamma-irradiation. In that earlier system, the region on the rod on which peptide was grafted was completely defined by the volume of reagent used for the coupling of specific amino acids to the growing peptide, or more accurately, the depth into which the rod dipped into the amino acid solutions. As this depth inevitably varied slightly from cycle to cycle in the synthesis, the result was a small region on the rod where the peptide synthesized may have had appreciable amounts of deletion peptides (that is, peptides whose sequence varied from that intended by having one or more residues absent) present because slightly less of one of the activated amino acid solutions was dispensed in one or more cycles.

A further disadvantage of the earlier system is that the polymer layer grafted onto the rods during the radiation process is readily solvated by many solvents and as a consequence, solvents will migrate upwards through this layer by capillary action. This results in depletion of the reservoir of reagent and consequently, as described above, a larger zone of uncertain synthesis quality is created. In addition, unless extreme care is taken in extensively washing these rods, the polymer layer acts as a reservoir of the solvent used in synthesis leading to the contamination of subsequent solutions.

According to one aspect of the present invention there is provided an object having a solid surface for use in the synthesis of peptides or other polymeric compounds thereon, said solid surface having first and second portions, said first surface portion having been treated to allow said synthesis to take place and said second surface portion having not been so treated, whereby said second surface portion is inert under the conditions of said synthesis, wherein the object has the shape of a rod, or pin, or geometric modification thereof.

According to a further aspect of the present invention there is provided a method for synthesizing peptides or other polymeric compounds on an object having a solid surface, which method comprises:
providing a synthesis surface on the object having a first portion treated to allow said synthesis and a second portion not so treated and which is inert under the conditions of said synthesis, the object being in the shape of a rod or pin or geometric modification thereof;
providing a reaction mixture for said synthesis comprising a reactant;
contacting said synthesis surface with said reaction mixture such that the entire first portion is in contact with said reaction mixture; and
coupling a reactant to said first portion.

In a first embodiment of the present invention, there is provided a surface for use in the synthesis of peptides or other polymeric compounds thereof, which surface comprises an active region on which said synthesis may take place and an inactive region on which said synthesis will not take place.

Preferably, the surface is the surface of a rod or pin of the general type disclosed in International Patent Specification No. PCT/AU84/00039, having a portion providing the active region and remaining portion providing the inactive region.

Preferably also, the portion of the rod or pin providing the active region of the surface on which the synthesis may take place is of a shape or construction which provides an increased ratio of surface area-to-volume in this active region.

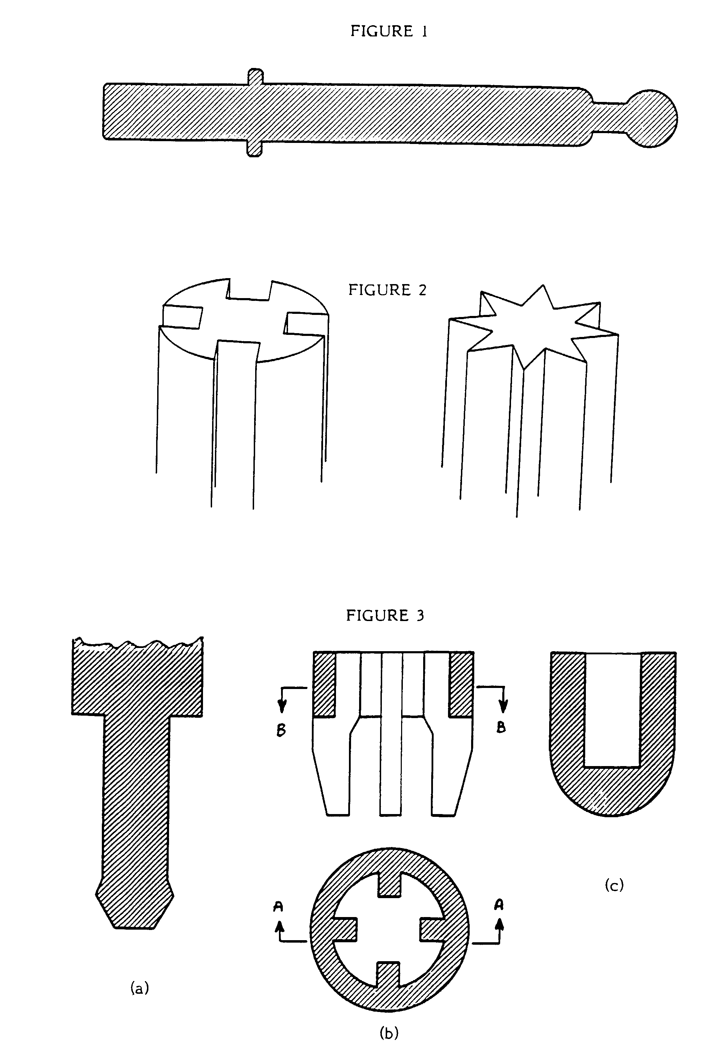

By way of illustration, Figure 1 represents a cross-sectional drawing a rod or pin. The 4.0 mm diameter sphere at one end will, after appropriate treatment, become the active region. This can be conveniently done by allowing only this region of the polyethylene pin to contact the acrylic acid solution during gamma-irradiation as disclosed in International Patent Specification No. PCT/AU84/00039. Thus, only this region of the pin will have polyacrylic acid grafted to it and consequently, only on this region of the pin or rod will synthesis be possible. Thus, the amount of peptide synthesized will be independent of the volume of reagents used during synthesis.so long as the depth of the solutions into which the rods or pins are immersed is sufficient to cover the active region during the coupling step. In this way, the possibility of deletion peptides will be reduced to a minimum. Furthermore, because the polyacrylic acid is grafted only on the active region, there is no possibility of solvent migration through capillary action as described above for the earlier system. Finally, because less polyacrylic acid has been grafted on to the surface of the rod or pin, the risk of contamination of subsequent solutions through "carry over" of solvents is correspondingly reduced.

In a second embodiment of the present invention there is provided a solid surface for use in the synthesis of peptides or other polymeric compounds thereon, which surface comprises an active region which is of a higher surface area/volume ratio than that of a cylinder of corresponding volume.

In another embodiment of the invention, the active region may be provided by a surface coating of a suitable resin applied to it. The coating can be made of any of the porous resins which are used for conventional solid phase peptide synthesis. Because these resins are porous, the surface area of the active region is increased dramatically and so allows a much greater yield of peptide or other polymer to be synthesized. In addition, the use of this embodiment of the invention makes it particularly convenient to change the chemistry used in the synthesis, and indeed, change the class of polymeric compounds to be synthesized by selecting the appropriate resin with which to coat the rod or pin. Examples of porous resins which may be used in such coatings include benzhydrylamine-polystyrene resin and polyacrylamide gel inside kieselguhr.

In a particularly preferred embodiment of the invention, the portion of the rod or pin providing the active region of the surface is manufactured as a separate entity, and then combined or assembled with the remaining portion of the rod or pin. Preferably, in this embodiment, the separately manufactured portion is a "tip" portion of the rod or pin, with the remaining portion being a "shaft" portion. This approach has two major advantages:

Firstly, the materials from which the portion providing the active region is manufactured may be selected mainly for their ability to act as a suitable base for peptide synthesis and only minimal regard given to other qualities such as mechanical strength. For instance, a soft, flexible plastic polymer can be chosen for the active region and the need to rigidly maintain the active region in a particular format can be disregarded. In another example, the portion providing the active region could be manufactured by sintering together spherical particles of glass, ceramic or other suitable materials. Furthermore, the remaining portion providing the inactive or support region may be constructed of a material selected solely for its mechanical properties and which may not be suitable at all for peptide synthesis. By way of example, this portion may be constructed from polypropylene or some other non-porous plastic material.

Secondly, the surface of the portion providing the active region will usually need to be treated to enable peptides or other polymers to be synthesized on to its surface. One suitable method disclosed in International Patent Specification No. PCT/AU84/00039 involves the grafting of polyacrylic acid onto the surface of a solid polyethylene support using gamma-irradiation. Dividing the rod into two functional portions - one providing the active region and another providing the inactive or support region also ensures that syntheses are more controlled. If the active region is the only component of the system which has been treated to allow synthesis of the peptide or other polymer, then synthesis will be restricted to it and it alone, in contrast to the earlier system, in which the complete rod was grafted with polyacrylic acid. In that earlier system, the region on the rod on which peptide was grafted was completely defined by the volume of reagent used for the coupling of specific amino acids to the growing peptide, or more accurately, the depth into which the rod dipped into the amino acid solutions. As this depth inevitably varied slightly from cycle to cycle in the synthesis, the result was a small region on the rod where the peptide synthesized may have had appreciable amounts of deletion peptides (that is, peptides whose sequence varied from that intended by having one or more residues absent) present because slightly less of one of the activated amino acid solutions was dispensed in one or more cycles. If the portion providing the active region is manufactured as a separate entity as described above, and its surface treated then only the active region will permit coupling of the amino acids or other monomers. Thus, the amount of peptide synthesized will be independent of the volume of reagents used during synthesis so long as the active region is covered by solution during the coupling step.

Another major advantage of manufacturing the portion providing the active region as a separate entity is the minimization of cross-contamination of solutions. The polymer layer grafted onto the rods during the radiation process as described in International Patent Specification No. PCT/AU84/00039 is readily solvated by many solvents and as a consequence, solvents will migrate upwards through this layer by capillary action. This results in depletion of the reservoir of reagent and consequently, as described above, a larger zone of uncertain synthesis quality is created. Also, unless extreme care is taken in extensively washing these rods, the polymer layer acts as a reservoir of the solvents used in synthesis leading to the contamination of subsequent solutions. Where the portion providing the active region is manufactured separately as described herein, this migration of solvents and reagents cannot take place.

Another major advantage of manufacturing the active region as a separate entity comes about because the active region will typically be much smaller than the complete unit. Therefore, more of the active region components can be treated simultaneously to create the active region with consequent savings in materials and time.

After treatment, the separately manufactured portions providing active regions (for example, tip portions) may be attached to the remaining portions (for example, shaft portions) by any suitable means. These include, but are not restricted to, heat fusing the active tip portion to the shaft portion; using a suitable adhesive to glue the active tip portion to the shaft portion; the mechanical attachment of the active tip portion to the shaft portion.

As previously described, it is preferred that the portion providing the active region be of a suitable shape or construction to provide an increased surface area/volume ratio in the active region. Such as increased surface area/volume ratio increases the amount of peptide or other polymer which can be synthesized when compared with, for example, a solid cylindrically shaped portion.

For the purpose of illustration only, the portion providing the active region of the rod, that is, the region of the rod on which the peptide or other polymeric molecule is to be synthesized, is a cylinder with a radius of 2 mm and a height of 4 mm, the surface area of which active region is 61.8 mm² (assuming that only one end of this cylinder is available for synthesis). However, if a slit lmm wide is made across the diameter of this portion of the rod, the surface area becomes 81.8 mm² (1.3 times the area of the solid cylindrical portion). The surface area of the portion providing the active region can be increased even further by modification of the shape of this portion of the rod. For instance, if eight slits, each 0.4 mm wide and lmm deep are made into the surface of a cylindrical portion, the surface area available for synthesis is increased to 124 mm² (twice the area of the unmodified cylindrical portion). It will be apparent that the surface area of the active region of the rod can be further increased by further modification of its geometric shape, and equally that such modifications can be made by molding the rod in its final desired shape or by machining a molded rod into its final desired shape.

However, in a preferred embodiment of the invention, the portion providing the active region of the rod is made by joining small particles of solid materials together, for instance, by sintering using pressure or heat or both. This can be particularly useful where it is desired to use particular harsh chemistries or virulent solvents. For instance, glass is resistant to most solvents which would make most conventional plastic materials unstable. Thus an active region could be made by sintering together small spherical beads of glass. This could then be treated, for instance, by functionalizing the surface with an amino-silane, to make it suitable as a base on which the peptide or other polymeric compound could be synthesized. In this example, the inactive region would be made from a particularly resistant plastic such as polytetrafluoroethylene. In this way, a material such as glass, which would be an unsuitable material for the inactive region, can be used with advantage in the active region.

A further advantage of this particular embodiment of the invention is the large increase in the surface area/volume ratio of the active region can be achieved. Using the example given above, 8885 spherical rigid particles each with a radius of 0.1 mm would occupy the volume of the portion providing the active region of the rod if close packed together. The surface area of these spheres would be 1116.4 mm², 17.8 times the surface area of a solid cylindrical portion. Decreasing the size of the particles to be sintered together, would provide a corresponding increase in the surface area available for synthesis. For example, decreasing the radius of the rigid spheres to be sintered to 0.055 mm increases the surface area to 2233 mm², about 35.5 times that of the solid cylinder. In practice, because of the process of joining the particles together, and the fact that the particles are neither uniform in size nor rigid, the theoretical increase in surface area would not be achieved. However, very significant gains in surface area available for synthesis can be achieved by making the solid support by sintering small particles of material together.

Further features of the present invention are illustrated, by way of example only, in the accompanying drawings. In Figure 2 there are shown two possible modifications which may be made to the geometric shape of the portion of the rod or pin providing the active region in order to increase the surface area of this active region. It will be appreciated that many other similar alterations of the geometric shape may be made to achieve a similar result.

Figure 3 illustrates, by way of example only, the two-part construction of a rod or pin of the general type disclosed in International Patent Specification No. PCT/AU84/00039. In this Figure (a) shows in longitudinal section the region of the "shaft" portion of the rod or pin on which is to be assembled the separately manufactured and treated "tip" portion. As will be seen from (a), this region is constructed so as to enable the tip portion to be retained on the narrowed section. The region shown in (a) is, of course, the end of the shaft portion of the rod or pin which in use will be remote from the holder in which the rods or pins are positioned. In Figure 3, (b) and (c) show two alternative configurations for the "tip" portion of the rod or pin which may be separately manufactured and treated to provide the active region. In (b), there are shown both the longitudinal section (along the line A-A) and cross section (along the line B-B) of one configuration which provides a "vaned" tip. In (c), there is shown in longitudinal section a more simple tip configuration which may, for example, be either a solid plastics tip or a molded tip made up of sintered small particles.

Further features of the present invention are illustrated, by way of example only, in the following example.

### EXAMPLE 1

10000 of the "vaned" tips illustrated in Figure 3(b) (with an external diameter of 5 mm) were gamma-irradiated in 1 litre of 6% acrylic acid solution as described in International Patent Specification No. PCT/AU84/00039. The tips then had hexamethylenediamine (HMD) coupled to the grafted polyacrylic acid by exposing them to a solution of 36.48 g t-butoxycarbonyl-hexamethylenediamine HCl, 24 ml triethylamine (TEA), 30.95 g dicyclohexylcarbodiimide (DCC), and 23.33 g 1-hydroxybenzotriazole (HOBt) in 1.2 L of dimethylformamide (DMF). The tips were then washed and deprotected using the usual methods. A proportion of the free amine groups on the tips were coupled to β-alanine by exposing the tips to a solution containing 26.19 g 9-fluorenylmethoxycarbonyl β-alanine, 17.07 g DCC, 25.27 g HOBt, and 3.56 g diisopropylethylamine in 1.37 L of DMF. After washing, the tips were assembled on their shafts and positioned in 12 x 8 matrices.

By contrast, 10000 of the rods or pins illustrated in Figure 1 were gamma-irradiated to graft polyacrylic acid to their entire surface using the earlier method disclosed in International Patent Specification No. PCT/AU84/00039. Because of the greater bulk of the complete rod or pins, 11 L of the acrylic acid solution was required. After grafting, the rods or pins were assembled in 12 x 8 matrices and HMD coupled to the spherical tip of the rods. 1.2 L of solution as described above was prepared and dispensed in 150 µL quantities into microtitre trays. The rods were then inserted into this solution. This quantity was sufficient to couple HMD to 7230 rods. After washing and deprotection in the usual manner, β-alanine was coupled to the free amine groups using 735 mL of the solution described above. This quantity was sufficient to couple 4608 rods or pins.

The amount of free amine groups on the tips after coupling with HMD was 2230 nmole/tip whereas it was 1015 nmole/pin for rods or pins treated in the earlier way as disclosed in International Patent Specification PCT/AU84/00039. This difference in free amine level reflected the greater surface area of the "vaned" tips. This example also illustrates that use of the invention allows more active regions to be made at one time without the need to use more reagents and without the need to individually dispense reagents.

The hexapeptide, EYYLNH (using the single letter code to represent the amino acid residues in the hexapeptide), was synthesized on to 8 of each the rods or pins described above. This synthesis was carried out using the standard Fmoc chemistry for solid-phase synthesis of peptides. The peptides were then assayed in a standard enzyme-linked immunosorbent assay (ELISA) using an antibody which specifically reacted with the peptide. The active regions synthesized according to the invention gave a mean absorbance of 2.55 with a standard deviation of 0.104 (coefficient of variance = 4%). By contrast, rods or pins treated in the earlier way as disclosed in International Patent Specification No. PCT/AU84/00039 gave a mean absorbance in the ELISA of 1.17 with a standard deviation of 0.116 (coefficient of variance = 10%).

This example illustrates the advantage of processing the active region of the rod or pin separately from the inactive region in substantially reducing rod-to-rod variation in testing.

## Claims

1. An object having a solid surface for use in the synthesis of peptides or other polymeric compounds thereon, said solid surface having first and second portions, said first surface portion having been treated to allow said synthesis to take place and said second surface portion having not been so treated, whereby said second surface portion is inert under the conditions of said synthesis, wherein the object has the shape of a rod, or pin, or geometric modification thereof.

2. The object of claim 1, wherein the geometry of the portion of the object providing said first surface portion is modified to provide a change in the surface area to volume ratio of the object at the first surface portion.

3. The object of claim 1 or 2, wherein the portion of the object providing said first surface portion is manufactured separately of the portion of the object providing said second surface portion and the two object portions are combined or assembled before use in the synthesis of peptides or other compounds.

4. The object of claim 3, wherein the portion of the object providing said first surface portion is manufactured by different processes or manufactured from different materials to the portion of the object providing said second surface portion.

5. The object of claims 3 or 4, wherein the portion of the object providing said first surface portion is formed by coalescing or sintering together a large number of smaller solid particles.

6. The object of any one of the preceding claims, wherein the portion of the object providing said first surface portion is of a higher surface area/volume ratio than that of a cylinder of corresponding volume.

7. The object of claim 6, wherein the portion of the object providing said first surface portion comprises a porous material.

8. The object of claim 6 or claim 7, wherein the portion of the object providing said first surface portion comprises sintered particles.

9. A method for synthesizing peptides or other polymeric compounds on an object having a solid surface, which method comprises:
providing a synthesis surface on the object having a first portion treated to allow said synthesis and a second portion not so treated and which is inert under the conditions of said synthesis, the object being in the shape of a rod or pin or geometric modification thereof;
providing a reaction mixture for said synthesis comprising a reactant;
contacting said synthesis surface with said reaction mixture such that the entire first portion is in contact with said reaction mixture; and
coupling a reactant to said first portion.

## Patentansprüche

1. Gegenstand, welcher eine feste Oberfläche zur Verwendung bei der Synthese von Peptiden oder anderen polymeren Verbindungen darauf besitzt, wobei die feste Oberfläche ein erstes und ein zweites Segment besitzt, wobei das erste Oberflächensegment in der Weise behandelt wurde, daß es das Stattfinden der Synthese gestattet, und das zweite Oberflächensegment nicht so behandelt wurde, wodurch das zweite Oberflächensegment unter den Synthesebedingungen inert ist, wobei der Gegenstand die Form eines Stabes oder einer Nadel oder einer geometrischen Modifikation davon besitzt.

2. Gegenstand nach Anspruch 1, dadurch **gekennzeichnet**, daß die Geometrie desjenigen Teils des Gegenstands, welcher das erste Oberflächensegment bereitstellt, in der Weise modifiziert ist, daß eine Änderung des Oberflächeninhalt/Volumen-Verhältnisses des Gegenstandes beim ersten Oberflächensegment erreicht wird.

3. Gegenstand nach Anspruch 1 oder 2, dadurch **gekennzeichnet**, daß derjenige Teil des Gegenstandes, welcher das erste Oberflächensegment bereitstellt, getrennt von demjenigen Teil des Gegenstandes, welcher das zweite Oberflächensegment bereitstellt, hergestellt wird, und die beiden Teile des Gegenstandes vor der Verwendung bei der Synthese von Peptiden oder anderen Verbindungen kombiniert oder zusammengesetzt werden.

4. Gegenstand nach Anspruch 3, dadurch **gekennzeichnet**, daß derjenige Teil des Gegenstandes, welcher das erste Oberflächensegment bereitstellt, durch andere Verfahren hergestellt wird oder aus anderen Materialien hergestellt wird als derjenige Teil des Gegenstandes, welcher das zweite Oberflächensegment bereitstellt.

5. Gegenstand nach den Ansprüchen 3 oder 4, dadurch **gekennzeichnet**, daß derjenige Teil des Gegenstandes, welcher das erste Oberflächensegment bereitstellt, dadurch gebildet wird, daß eine große Zahl kleinerer fester Partikel verschmolzen oder zusammengesintert wird.

6. Gegenstand nach einem der vorstehenden Ansprüche, dadurch **gekennzeichnet**, daß derjenige Teil des Gegenstands, welcher das erste Oberflächensegment bereitstellt, ein größeres Oberflächeninhalt/Volumenverhältnis als ein Zylinder mit vergleichbarem Volumen besitzt.

7. Gegenstand nach Anspruch 6, dadurch **gekennzeichnet**, daß derjenige Teil des Gegenstandes, welcher das erste Oberflächensegment bereitstellt, ein poröses Material umfaßt.

8. Gegenstand nach Anspruch 6 oder Anspruch 7, dadurch **gekennzeichnet**, daß derjenige Teil des Gegenstandes, welcher das erste Oberflächensegment bereitstellt, gesinterte Partikel umfaßt.

9. Verfahren zur Synthese von Peptiden oder anderen polymeren Verbindungen auf einem Gegenstand, welcher eine feste Oberfläche besitzt, wobei das Verfahren folgendes umfaßt:
Bereitstellen einer Syntheseoberfläche auf dem Gegenstand, welche ein erstes Segment, welches in der Weise behandelt wurde, daß es die Synthese gestattet, und ein zweites Segment, welches nicht so behandelt wurde und welches unter den Synthesebedingungen inert ist, besitzt, wobei der Gegenstand in Form eines Stabes oder einer Nadel oder einer geometrischen Modifikation davon vorliegt;
Bereitstellen eines Reaktionsgemisches für die Synthese, umfassend einen Reaktanten;
Inkontaktbringen der Syntheseoberfläche mit dem Reaktionsgemisch in der Weise, daß das gesamte erste Segment in Kontakt mit dem Reaktionsgemisch ist; und
Kuppeln eines Reaktanten an das erste Segment.

## Revendications

1. Objet ayant une surface solide pour son utilisation dans la synthèse de peptides ou d'autres composés polymèriques, ladite surface solide ayant des première et seconde parties, ladite première partie de surface ayant été traitée pour permettre à ladite synthèse de se produire et ladite seconde partie de surface n'ayant pas été ainsi traitée, de sorte que ladite seconde partie de surface est inerte dans les conditions de ladite synthèse, l'objet ayant la forme d'une tige, d'une broche ou d'une modification géométrique de celles-ci.

2. Objet selon la revendication 1, dans lequel la géométrie de la partie de l'objet fournissant ladite première partie de surface est modifiée pour fournir un changement du rapport aire de surface à volume de l'objet à la première partie de surface.

3. Objet selon la revendication 1 ou 2, dans lequel la partie de l'objet fournissant ladite première partie de surface est fabriquée séparément de la partie de l'objet fournissant ladite seconde partie de surface, et les deux parties de l'objet sont combinées ou assemblées avant usage dans la synthèse de peptides ou d'autres composés.

4. Objet selon la revendication 3, dans lequel la partie de l'objet fournissant ladite première partie de surface est fabriquée par différents procédés ou fabriquée à partir de différentes matières par rapport à la partie de l'objet fournissant ladite seconde partie de surface.

5. Objet selon la revendication 3 ou 4, dans lequel la partie de l'objet fournissant ladite première partie de surface est formée en coalescant ou en frittant ensemble un grand nombre de particules solides plus petites.

6. Objet selon l'une quelconque des revendications précédentes, dans lequel la partie de l'objet fournissant ladite première partie de surface a un rapport aire de surface/volume plus élevé que celui d'un cylindre d'un volume correspondant.

7. Objet selon la revendication 6, dans lequel la partie de l'objet fournissant ladite première partie de surface comprend une matière poreuse.

8. Objet selon la revendication 6 ou la revendication 7, dans lequel la partie de l'objet fournissant ladite première partie de surface comprend des particules frittées.

9. Procédé pour synthétiser des petites ou d'autres composés polymèriques sur un objet ayant une surface solide, ce procédé comprenant :
la fourniture d'une surface de synthèse sur l'objet ayant une première partie traitée pour permettre ladite synthèse et une seconde partie non ainsi traitée et qui est inerte dans les conditions de ladite synthèse, l'objet étant sous la forme d'une tige ou d'une broche ou d'une modification géométrique de celles-ci ;
la fourniture d'un mélange réactionnel comprenant un réactif pour ladite synthèse ;
la mise en contact de ladite surface de synthèse avec ledit mélange réactionnel de sorte que la totalité de la première partie est en contact avec ledit mélange réactionnel ; et
le couplage d'un réactif à ladite première partie.
